# EUROPEAN PATENT APPLICATION

(11) **EP 2 277 581 A1**
(43) Date of publication of application: **26.01.2011**
(21) Application number: 10170158.9
(22) Date of filing: 20.07.2010
(51) Int. Cl.: A61M 25/02

(54) **A device for fastening and protecting a catheter unit**

(30) Priority: 20.07.2009 DK 200970072
(71) Applicant: Ostergaard, Hans Jorgen Lund, 7100 Vejle (DK)
(72) Inventor: Østergaard, Hans Jørgen Lund, 7100, Vejle (DK); Lange, Per Jørn, 2860, Søborg (DK)
(74) Representative: Plougmann & Vingtoft A/S

(57) **Abstract**

The invention relates to a device for fastening and protecting a catheter unit (10) of the type comprising transversely extending locking flaps (27) and a transversely extending neck or web (31). Such device (17) comprises a sheet or base plate member (18) defining a longitudinally extending slit or slot (23), which opens at a first end of the plate member for receiving the neck or web (31) of the catheter unit (10) therein so as to interlock the catheter unit and the base plate member. The base plate member has means (21) for fastening a first side surface thereof in contact with the skin surface of a patient. A cover member (19) is hinge connected to or slide-able in relation to the base plate member so as to be movable between open and closed positions. In the closed position of the cover member a housing for containing a substantial part of the catheter unit (10) is defined between the base plate member (18) and the cover member (19).

## Description

The present invention relates to a device for fastening and protecting a catheter unit of the type comprising a catheter shaft, a needle extending longitudinally therefrom, and transversely extending locking flaps connected to the shaft by a connecting neck or web. It is general practise to insert a catheter, for example a co-called "IV catheter", into a vein in the hand or forearm of a hospitalised person. The purpose is to allow infusion of a therapeutic liquid and/or injection of a medicament directly into the vein. A doctor or a nurse inserts the catheter into the vein whereafter a catheter shaft or handle of the catheter unit is fastened to the skin of the patient by means of adhesive tape. A liquid inlet at the free end of the catheter shaft may be connected to one end of a hose, while the other end of the hose may be connected to a container for an infusion liquid. This means that a 180° bend must be formed on the hose, which is kept in this position also by means of adhesive tape. However, care must be taken to prevent the hose from kinking. This fastening procedure may vary from person to person performing it. Further, the catheter unit may comprise projections in the form of injection ports, finger grips, etc., which may tend to become entangled with bedclothes, clothes and other items worn by the patient, when the patient moves, whereby the catheter unit may become dislocated.

However, catheter fastening and protecting devices are known, vide for example US 4,275,721, US 5,112,313, US 5,449,349, US 6,827,707 B2, US 2003/0229313 A2, US 2004/0204685 A1, US 2005/0215953 A1, US 2007/0043326 A1, GB 1535899 A1, WO92/12757 A1, WO 2007/024900 A2, W02007/082093 A2.

These known units have not been used in practice to any substantial extent, probably because they are rather expensive to produce and/or complicated to use. Thus, the object of the present invention is to provide a catheter fastening and protecting device which is relatively inexpensive to produce and easy to install or mount.

It is also known to fasten a catheter unit of the above type by means of a flexible plastic sheet member defining an inwardly tapering slit therein. The catheter unit may then be fastened to a patient's skin surface by applying the sheet member such that the said connecting neck of the unit extends through the slit while the locking flaps are sandwiched between the sheet member and the skin surface when the sheet member is fastened to the skin surface by means of an adhesive. This sheet member, which is commonly used, does not protect the catheter unit from being entangled with clothes and other items worn by the patient, whereby the unit may become dislocated.

The present invention provides a device for fastening and protecting a catheter unit of the type comprising a catheter shaft, a needle extending longitudinally therefrom, and transversely extending locking flaps connected to the shaft by a connecting neck, said device comprising a sheet or base plate member defining a longitudinally extending slit or slot opening at a first end of the plate member for receiving the connecting neck of the catheter unit therein so as to interlock the catheter unit and the base plate member, said base plate member having means for fastening a first side surface thereof in contact with a skin surface, and the device according to the invention is characterized in further comprising a cover member, which is movable between an open position, in which a second side surface of the base plate member opposite to said first side surface is substantially uncovered, and a closed position in which a housing for containing a substantial part of the catheter unit is defined between the base plate member and the cover member. Such a device, which may be produced relatively inexpensive, is able not only to efficiently lock a catheter unit to a skin surface, but also to prevent the catheter unit from becoming entangled with bedclothes, patient's clothes or other items and thereby become dislocated when the patient is moving. When a doctor or nurse has inserted the catheter needle into a vein of the patient a shaft or handle of the catheter unit may be fastened to the skin of the patient by positioning the base plate member above the locking flaps and with said connecting neck within the slit or slot. Thereafter the base plate member - and consequently also the catheter fastening and protecting device - is fastened to the skin of the patient, preferably by means of an adhesive.

The cover member may be an integral part of the base plate member. As an example the cover member may be hinge connected to the base plate along a first longitudinal edge part thereof, and releasable locking means for locking the cover member in its closed position may be arranged at an opposite longitudinal second edge part of the base plate member. The whole device may then be formed integrally from a flexible sheet material, such as plastics material, in a single operation. The cover member may be formed with a curved cross-section, or the cover member may be substantially flat. In the latter case the width of the flexible cover member may substantially exceed that of the base plate member, so that the cover member is curved when it is moved to and locked in its closed position.

Alternatively, the base plate member and the cover member may be separate parts, and releasable locking means for locking the cover member in its closed position may be arranged at both of the first and second edge parts of the base plate member. Also in this case the cover member may be formed with a curved cross-section, or the cover member may be substantially flat and flexible, so that the cover member is being curved when it is moved to and locked in its closed position. The releasable locking means may be of any type, such as a type of snap fastener or catch. In its most simple embodiment the locking means may comprise a projection or flange on the cover member engaging beneath a projection or flange formed at a longitudinal edge part of the base plate member.

When the base plate member and the cover member are separate parts the cover member may be longitudinally displace able in relation to the base plate member between said open and closed positions. Thus, the cover member may function as a sliding lid. This may be obtained for example when inter-engaging guide members, such as flanges, are formed along the longitudinal edge portions of the base plate member and/or the cover member.

The fastening and protecting device according to the invention may be adapted to interlock a catheter unit of the type having an injection port. In that case the wall part of the cover member covering the injection port may be penetrable by a catheter or needle. Alternatively, an opening or cut-out may be formed in the cover member opposite to the injection port in the closed position of the cover member, so that the injection port is accessible even when the cover member is in its closed position. This opening or cut-out may be covered by a lid, which may, for example, be hinge-connected to the cover member.

The catheter may be an infusion catheter, which is connected to an infusion container via a flexible tubing or hose. In such case a bend of about 180° usually has to be formed on the hose. In order to retain the hose in the desired position the fastening and protecting device preferably comprises means formed on said second side surface of the base plate member for holding a hose, which means are connected to the catheter unit. Said holding means may comprise a channel for resiliently receiving the hose or a hook- or clamp-like member extending upwardly from said second side surface.

The device according to the invention may be made from any suitable material, such as reinforced cardboard, but the base plate member as well as the cover member is preferably made from plastics material.

The outer surface of the fastening and protecting device may have a shape without transversely extending projections, whereby the risk that parts of the device is unintentionally caught by clothing or other items worn by the patient is substantially reduced or eliminated.

Non-limiting embodiments of he invention will now be further described with reference to the drawings, wherein
- Fig. 1: illustrates a conventional infusion process,
- Fig. 2: is a top plan view of an embodiment of the fastening and protecting device according to the invention,
- Fig. 3: is an end view of the device shown in Fig. 2,
- Fig. 4: is a top plan view of a conventional catheter unit fastened to the skin of a patient by means of the fastening device shown in Figs. 2 and 3,
- Fig. 5: is a cross-sectional view of a further embodiment of the fastening and protecting device mounted on the skin surface of an arm or a hand of a patient,
- Fig. 6: is a perspective view of an amended embodiment, and
- Fig. 7: is a bottom view of the device shown in Fig. 6.

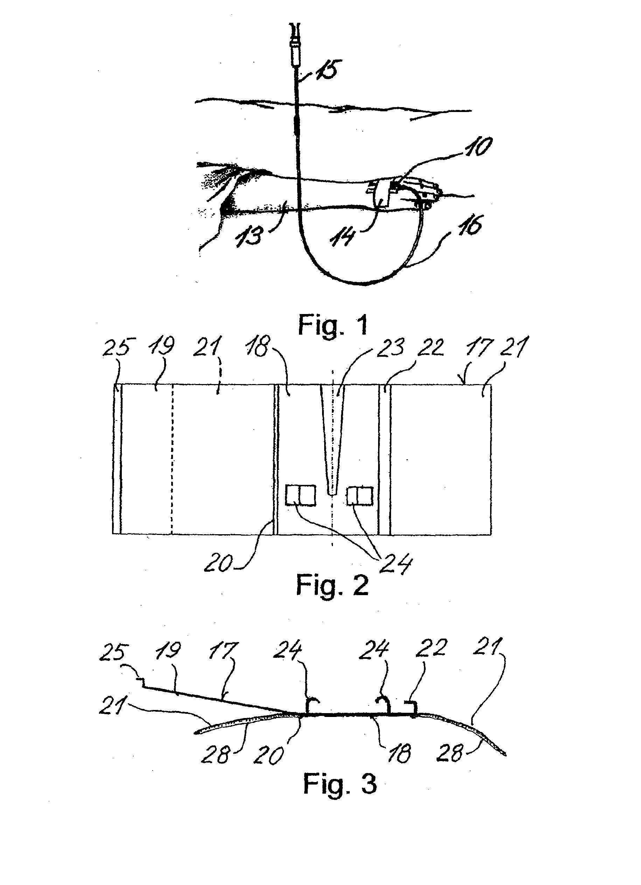

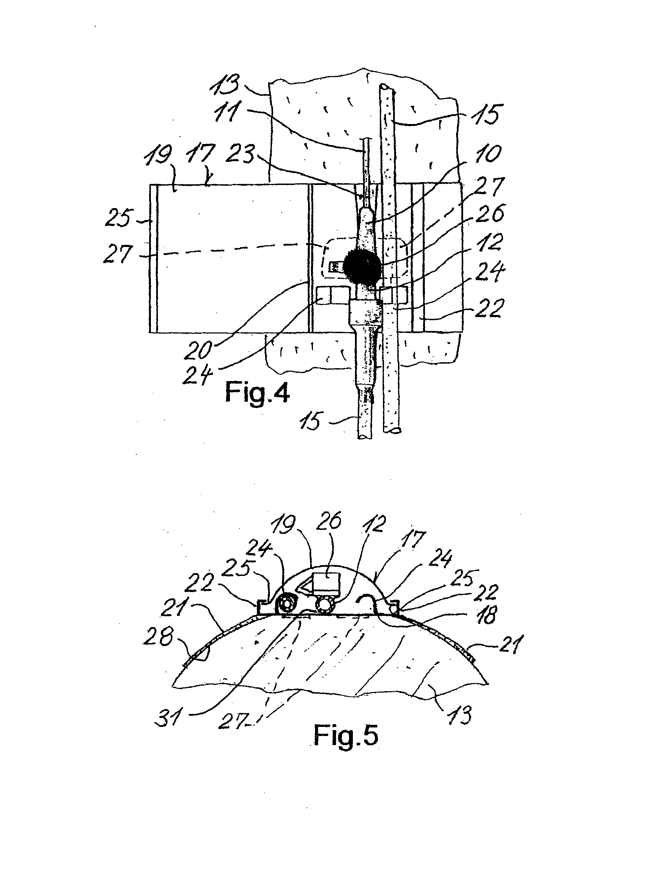

A conventional catheter unit 10 includes a catheter or needle 11 and a catheter shaft 12 (vide Fig. 4). Fig. 1 illustrates an infusion process, where the catheter needle of a conventional catheter unit, such as a "IV catheter" has been inserted into a vein in a hand or forearm 13 of a patient. In a conventional manner the catheter shaft 12 has been fastened to the skin of the patient by means of an adhesive tape 14. The catheter shaft is connected to an infusion container (not shown) by means of a flexible hose or tubing 15. As shown in Fig. 1 a bend 16 of about 180° or more has to be formed on the hose 15 and usually also the hose is fastened to the forearm 13 of the patient by means of adhesive tape.

The device 17 shown in Figs. 2-4 comprises a base plate member 18 and a cover member or lid 19 connected to the base plate member 18 along a hinge line 20 which defines a longitudinal side of the base plate member. The device 17 further comprises a pair of fastening sheet members 21 which are connected to the base plate member 18 along its opposite longitudinal sides. An upstanding channel-like edge part 22 is formed along the longitudinal side of the base plate member 18 opposite to the hinge line 20. A slit or slot 23 extends longitudinally from an open end towards a closed end and the width of the slot is decreasing towards its closed end. Furthermore, a pair of hook-like holding members 24 for releasably holding a hose or tubing 15 is extending from the upper side of the base plate member 18. A locking projection or flange 25 for engaging with the edge part 22 of the base plate member 18 is formed along the free longitudinal edge of the cover member 19.

When a catheter needle 11 of a catheter unit 10, such as a "IV catheter", has been inserted into a vein in a hand or forearm 13 of a patient the fastening device 16 may be slid over the catheter unit 10 such that a neck or web 31 (Fig. 5) interconnecting the catheter shaft 12 and locking flaps 27 of the catheter unit extends transversely through the slit 23 while the locking flaps 27 are sandwiched between the base plate member 18 and the skin surface of the patient. The injection port 26 may be used for injection of a medicament into the infusion liquid by means of a syringe, not shown. In such case the cover member or lid 19 may comprise an opening or cut-out (not shown) through which the injection port is accessible. The said opening or cut-out may be covered by a further lid or cover movable between open and closed positions.

The bottom side of the fastening sheet members 21 and possibly also of the base plate member may be covered by an adhesive layer 28 and a protective sheet. When the protective sheet has been removed the sheet members 21 are pressed against the skin surface of the patient so as to fasten the base plate member 18 of the protective device 17 in close contact with the skin surface of the patient. Thereafter a 180° bend may be formed on the hose or tubing 15 and a part thereof may be inserted in one of the hook shaped holding members 24. Now, the cover member 19 are turned around the hinge 20 and compressed transversely so that the locking projection or flange 25 of the cover member 19 may snap into locking engagement with the channel-like edge part 22 of the base plate member 18. Because the cover member 19 is wider than the base plate member 18 the cover member is upwardly curved in the closed position. Thus, a chamber for housing and protecting the major part of the catheter unit 10 is formed between the base plate member 18 and the cover member 19.

Fig. 5 is a cross-sectional view of a further embodiment of the fastening and protecting device according to the invention shown in its position of use. In the following description of Figs. 5-7 parts corresponding to similar parts of the embodiment of Figs. 1-4 will designated the same reference numerals.

In Fig. 5 the base plate member 18 and the cover member 19 are separate parts, and the hinge line 20 in the embodiment of Figs. 1-4 has been replaced by a locking projection or flange 25 which may engage with a corresponding channel-like edge part 22 formed on the base plate member 18, so that the cover member 19 may function as a sliding lid. In other respects the embodiment of Fig. 5 corresponds to that of Figs. 1-4. The cover member 19 may be relatively stiff and formed with a cross-section like a segment of a circle or another curved cross-section. Alternatively, the cover member may be flexible and flat in its open position and may then be compressed in to its curved shape when mounted in engagement with the oppositely arranged edge portions 22 of the base plate member 18.

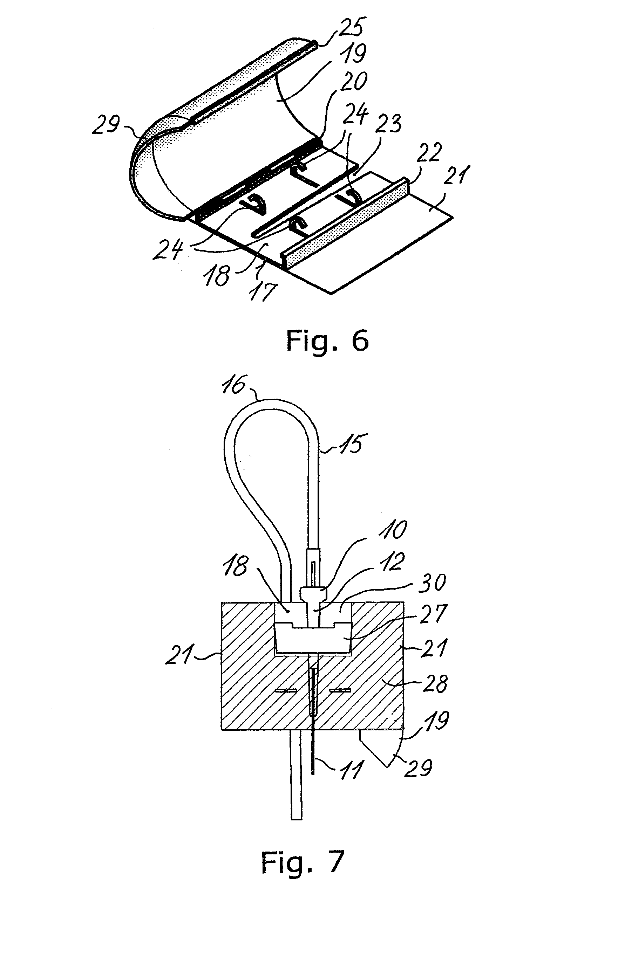

Figs. 6 and 7 illustrate a further embodiment, in which the lid 19 has a permanently curved cross-sectional shape and is connected to the base plate member 18 along the hinge 20. At the end adjacent to the closed end of the slit 23 the lid 19 has an end portion 29 extending beyond the base plate member 18 in the closed position of the lid. Preferably, this end portion 29 has a radius of curvature decreasing towards its free end as illustrated in Fig. 6.

In Fig. 7 the part of the bottom surface of the base plate member 18 which has been provided with the adhesive layer 28 has been indicated by hatching. Thus, a rectangular area 30 for engaging with the locking flaps 27 has not been covered by the adhesive. As shown in Fig. 7 the catheter unit 10 need not be arranged as shown in Fig. 4 where the needle extends through the open end of the slit or slot 23 in the base plate member 18, but could alternatively be arranged such that the needle extends in the opposite direction. However, in both cases the locking flaps 27 are sandwiched between the bottom surface of the base plate member 18 and the skin of the patient, and the neck or web 31 extends transversely thereto through the narrowing slit 23 in the base plate member 18.

It should be understood that numerous modifications and changes of the embodiments described above could be made within the scope of the present invention as defined by the following claims.

## Claims

1. A device for fastening and protecting a catheter unit (10) of the type comprising a catheter shaft (12), a needle (11) extending longitudinally therefrom, and transversely extending locking flaps (27) connected to the shaft by a connecting neck or web (31), said device (17) comprising a sheet or base plate member (18) defining a longitudinally extending slit or slot (23) opening at a first end of the plate member for receiving the connecting neck of the catheter unit (10) therein so as to interlock the catheter unit and the base plate member, said base plate member having means (21) for fastening a first side surface thereof in contact with a skin surface, **characterized in** further comprising a cover member (19), which is movable between an open position, in which a second side surface of the base plate member (18) opposite to said first side surface is substantially uncovered, and a closed position in which a housing for containing a substantial part of the catheter unit (10) is defined between the base plate member (18) and the cover member (19).

2. A device according to claim 1, wherein the cover member (19) is hinge connected to the base plate member (18) along a first longitudinal edge part (20) thereof, releasable locking means (22) for locking the cover member (19) in its closed position being arranged at an opposite longitudinal second edge part of the base plate member (18).

3. A device according to claim 1 or 2, wherein the base plate member (18) and the cover member (19) are formed in one piece.

4. A device according to claim 1, wherein the base plate member (18) and the cover member (19) are separate parts, releasable locking means (22) for locking the cover member (19) in its closed position being arranged at a first and second longitudinal edge part of the base plate member (18).

5. A device according to claim 1, wherein the base plate member (18) and the cover member (19) are separate parts, the cover member (19) being longitudinally displaceable in relation to base plate member (18) between said open and closed positions.

6. A device according to claim 5, wherein inter-engaging guide members (22, 25) are formed along the longitudinal edge portions of the base plate member (18) and/or the cover member (19).

7. A device according to any of the claims 1 - 6 and adapted to interlock a catheter unit (10) of the type having an injection port (26), an opening or cut-out being formed in the cover member opposite to the injection port in the closed position of the cover member (19).

8. A device according to any of the claims 1 - 7, wherein means (24) for holding a hose or tubing (15), which is connected to the catheter unit (10), are formed on said second side surface of the base plate member (18).

9. A device according to claim 8, wherein said holding means comprise a hook- or clamp-like member (24) extending from said second side surface.

10. A device according to any of the claims 1 - 9, wherein the base plate member (18) and the cover member (19) are made from plastics material.
